# EUROPÄISCHE PATENTANMELDUNG

(11) **EP 4 223 324 A1**
(43) Veröffentlichungstag der Anmeldung: **09.08.2023**
(21) Anmeldenummer: 23152529.6
(22) Anmeldetag: 19.01.2023
(51) Int. Cl.: A61L 9/04, A61L 9/12, B65D 5/38, B65D 5/42, B65D 5/56, A01M 1/20, B32B 29/00

(54) **VORRICHTUNG MIT EINEM GEHÄUSE AUS EINEM MINDESTENS DREISCHICHTIGEN MATERIAL ZUR ABGABE EINES FLÜCHTIGEN STOFFS AN DIE UMGEBUNGSLUFT**

(30) Priorität: 07.02.2022 DE 102022201257
(71) Anmelder: Swif GmbH South West International Fragrance, 67661 Kaiserslautern (DE)
(72) Erfinder: KARG, Jörn, 67661 Kaiserslautern (DE)
(74) Vertreter: Prescher, Gordian

(57) **Zusammenfassung**

Die vorliegende Erfindung betrifft eine Vorrichtung (1) zur Abgabe eines flüchtigen Stoffs an die Umgebungsluft, mit einem Gehäuse (10) aus einem ersten, mindestens dreischichtigem Material und mit einer innerhalb des Gehäuses (10) angeordneten Einlage (20) aus einem zweiten Material, wobei das zweite Material mit dem flüchtigen Stoff versehen, insbesondere imprägniert, ist, wobei das zweite Material eines der folgenden Materialien ist eines oder mehrere der genannten Materialien aufweist:
- ein pflanzlicher Faserstoff oder
- Saccharose oder Stärke oder
- ein mineralisches Material.

## Beschreibung

### Stand der Technik

Die vorliegende Erfindung betrifft eine Vorrichtung zur Abgabe eines flüchtigen Stoffs, insbesondere Duftstoffs oder insektiziden Wirkstoffs, an die Umgebungsluft mit einem Gehäuse, welches ein mindestens dreischichtiges erstes Material aufweist und wobei eine erste Schicht ein Papier oder einen Karton oder eine Pappe enthält oder daraus besteht, eine zweite Schicht Polyethylen (PE) enthält oder daraus besteht und eine dritte Schicht orientiertes Polypropylen (z.B. (B)OPP) enthält oder daraus besteht.

Ähnliche Vorrichtungen sind beispielsweise aus US 4 258 874 A, US 4 166 565 A und US 4 279 373 A bekannt und weisen ein Gehäuse auf, das beispielsweise aus einem Papier oder Karton ausgebildet ist. Innerhalb des Gehäuses ist typischerweise eine Abgabeeinrichtung angeordnet, die während des Gebrauchs der Vorrichtung einen flüchtigen Stoff, z. B. einen Duftstoff, abgeben kann. Bei diesen Vorrichtungen hat es sich als nachteilig erwiesen, dass der flüchtige Stoff bereits vor der eigentlichen Verwendung der Vorrichtung durch das Gehäuse aus Papier oder Pappe diffundieren kann. Hierdurch steht dann während des Benutzungsintervalls der Vorrichtung nur eine reduzierte Menge des flüchtigen Stoffs zur Verfügung. Ein weiterer Nachteil besteht darin, dass nach dem Gebrauch der Vorrichtung typischerweise Rückstände in dem Gehäuse verbleiben, die nicht biologisch abbaubar sind. Hierdurch wird die Entsorgung bzw. das Recycling derartiger Vorrichtungen erschwert.

Eine weitere Vorrichtung zur Abgabe eines Duftstoffs ist aus der US 4 712 737 A bekannt. Diese Vorrichtung umfasst ein Gehäuse aus beschichtetem Karton sowie eine innerhalb des Gehäuses angeordnete Abgabevorrichtung, die beispielsweise aus einem Gelsystem, einem Gewebe oder einem Vlies gebildet sein kann. Auch bei dieser Vorrichtung können nach dem Gebrauch nicht biologisch abbaubare Rückstände im Gehäuse verbleiben.

Die Deutsche Patentanmeldung Nr. 10 2020 120 951.7 offenbart eine Vorrichtung zur Abgabe eines flüchtigen Stoffs, insbesondere Duftstoffs oder insektiziden Wirkstoffs, an die Umgebungsluft.

### Offenbarung der Erfindung

Eine Aufgabe der vorliegenden Erfindung ist es, die Entsorgung bzw. das Recycling einer Vorrichtung zur Abgabe eines flüchtigen Stoffs an die Umgebungsluft zu erleichtern.

Zur Lösung der Aufgabe wird eine Vorrichtung zur Abgabe eines flüchtigen Stoffs, insbesondere eines Duftstoffs oder eines insektiziden Wirkstoffs, an die Umgebungsluft vorgeschlagen, mit
**a)** einem Gehäuse (10), welches
   - ein mindestens dreischichtiges erstes Material enthält und wobei
      (i) eine erste Schicht des mindestens dreischichtigen ersten Materials ein Papier oder ein Karton oder eine Pappe enthält oder daraus besteht, und
      (ii) eine zweite Schicht des mindestens dreischichtigen ersten Materials Polyethylen (PE) enthält oder daraus besteht, und
      (iii) eine dritte Schicht des mindestens dreischichtigen ersten Materials orientiertes Polypropylen (z.B. (B)OPP) enthält oder daraus besteht und mindestens diese dritte Schicht eine Barrierefunktion gegen die Permeation des flüchtigen Stoffs aufweist, und
   - ggfs. eine oder mehrere zusätzliche Barriereschichten mit einer Barrierefunktion gegen die Permeation des flüchtigen Stoffs aufweist,
   und
**b)** einer innerhalb des Gehäuses (10) angeordneten Einlage (20) aus einem zweiten Material, wobei das zweite Material mit dem flüchtigen Stoff versehen, insbesondere imprägniert, ist, wobei das zweite Material eines der folgenden Materialien ist oder eines oder mehrere der genannten Materialien aufweist:
   - ein pflanzlicher Faserstoff oder
   - Saccharose oder Stärke oder
   - ein mineralisches Material.

Derartige Vorrichtungen können auch als Raumlufterfrischer oder Insektenvernichter oder Insektenrepellent bezeichnet werden.

Die erfindungsgemäße Vorrichtung umfasst ein Gehäuse aus einem ersten Material. Dieses erste Material weist eine erste Schicht enthaltend oder bestehend aus Papier, Karton oder Pappe, eine zweite Schicht enthaltend oder bestehend aus Polyethylen (PE) und eine dritte Schicht enthaltend oder bestehend aus orientiertem Polypropylen (z.B. (B)OPP) auf. Ein weiterer Bestandteil der Vorrichtung ist eine Einlage aus einem zweiten Material, welches sich von dem ersten Material vorzugsweise unterscheidet. Somit können das erste und zweite Material spezifisch für die jeweilige Funktion ausgewählt werden. Denn während das erste Material des Gehäuses vor der Benutzung der Vorrichtung eine Barrierefunktion für den flüchtigen Stoff bilden muss, sollte das zweite Material möglichst gute für die Abgabe des flüchtigen Stoffs relevante Eigenschaften haben. Das zweite Material ist ein pflanzlicher Faserstoff oder Saccharose oder Stärke oder ein mineralisches Material. Das zweite Material ist mit dem flüchtigen Stoff versehen, insbesondere imprägniert, so dass dieser aus dem Gehäuse an die Umgebungsluft abgegeben werden kann. Auch die Einlage der Vorrichtung ist nach dem Gebrauch ohne großen Aufwand zu entsorgen bzw. zu recyclen. Es ist möglich, das Gehäuse und die Einlage gemeinsam, d.h. ohne sie trennen zu müssen, zu entsorgen, z.B. im Papiermüll oder Verpackungsmüll ("gelber Sack").

Erfindungsgemäß enthält oder beinhaltet die erste Schicht (typischerweise "äußere Schicht") des ersten Materials Papier, Karton oder Pappe, insbesondere Karton, d.h. vorzugsweise einen spezifischen pflanzlichen Faserstoff. Eine "äußere Schicht" ist vorzugsweise eine solche Schicht, welche bei Betrachtung der fertiggestellten Vorrichtung zumindest teilweise zu erkennen ist, ohne die fertiggestellte Vorrichtung auseinanderbauen zu müssen. Sofern mehrere Schichten bei Betrachtung der fertiggestellten Vorrichtung zumindest teilweise zu erkennen sind, ohne die fertiggestellte Vorrichtung auseinanderbauen zu müssen, weist die Vorrichtung vorzugsweise mehrere "äußere Schichten" auf. Vorzugsweise kann eine "äußere Schicht" als eine solche Schicht aufgefasst werden, welche von der Einlage räumlich abgewandt ist. Vorzugsweise ist die erste Schicht des ersten Materials, welche Papier, Karton oder Pappe enthält oder daraus besteht, eine "äußere Schicht".

Erfindungsgemäß dient die zweite Schicht des ersten Materials, welche Polyethylen (PE) enthält oder daraus besteht, als Haftvermittler zwischen der ersten Schicht des ersten Materials und der dritten Schicht des ersten Materials. Bevorzugte Polyethylene umfassen Low-Density Polyethylen (LDPE / PE-LD), Linear-Low-Density Polyethylen (LLDPE / PE-LLD) und/oder High-Density Polyethylen (HDPE / PE-HD). Mit "LDPE" wird Polyethylen niedriger Dichte bezeichnet, welches eine Dichte im Bereich von z.B. 0,86 bis 0,93 g/cm³ aufweisen kann und sich durch einen hohen Verzweigungsgrad der Moleküle auszeichnet. Mit "HDPE" wird Polyethylen hoher Dichte bezeichnet, welches nur eine geringe Verzweigung der Molekülkette aufweist, wobei die Dichte im Bereich von z.B. 0,94 bis 0,97 g/cm³ liegen kann.

Erfindungsgemäß dient die dritte Schicht (typischerweise "innere Schicht") des ersten Materials, welche orientiertes Polypropylen (z.B. (B)OPP) enthält oder beinhaltet, als Barriereschicht. Das Polypropylen kann z.B. in einer Quer- oder Längsrichtung (monoaxial = OPP) oder auch in beiderlei Richtungen (biaxial = BOPP) gestreckt sein. Diese Schicht weist hervorragende Barriere-Eigenschaften gegen beispielsweise Gerüche, Aromen, Gase und Wasserdampf auf. Vorzugsweise ist die dritte Schicht des ersten Materials wasserabweisend und/oder zumindest teilweise acrylbeschichtet. Ferner weist diese Barriereschicht eine Barrierefunktion gegen die Permeation des flüchtigen Stoffs auf. Unter einer solchen Barriereschicht wird eine Schicht verstanden, die eine geringe Permeabilität für den flüchtigen Stoff aufweist. Durch die Barriereschicht kann verhindert werden, dass der flüchtige Stoff vor einer Aktivierung der Vorrichtung aus dem Gehäuse entweichen kann. Durch die Barriereschicht kann im Inneren des Gehäuses ein derart hoher Partialdruck des flüchtigen Stoffs aufrechterhalten werden, dass ein weiteres Ausdiffundieren des flüchtigen Stoffs aus der Einlage vermieden wird. Eine "innere Schicht" ist vorzugsweise eine solche Schicht, welche bei Betrachtung der fertiggestellten Vorrichtung zumindest teilweise nicht zu erkennen ist, ohne die fertiggestellte Vorrichtung auseinanderbauen zu müssen. Sofern mehrere Schichten bei Betrachtung der fertiggestellten Vorrichtung zumindest teilweise nicht zu erkennen sind, ohne die fertiggestellte Vorrichtung auseinanderbauen zu müssen, weist die Vorrichtung vorzugsweise mehrere "innere Schichten" auf. Vorzugsweise kann eine "innere Schicht" als eine solche Schicht aufgefasst werden, welche der Einlage räumlich zugewandt ist. Vorzugsweise ist die dritte Schicht des ersten Materials, welche orientiertes Polypropylen (z.B. (B)OPP) enthält oder beinhaltet, eine "innere Schicht".

Geeignetes Papier, Karton, Pappe, Polyethylen (PE) und orientiertes Polypropylen (z.B. (B)OPP) sind einem Fachmann grundsätzlich bekannt und kommerziell erhältlich.

Die (i) erste Schicht des mindestens dreischichtigen ersten Materials, die (ii) zweite Schicht des mindestens dreischichtigen ersten Materials und/oder die (iii) dritte Schicht des mindestens dreischichtigen ersten Materials können, wenn notwendig, jeweils unabhängig voneinander, einen oder mehrere Zusatzstoffe ausgewählt aus der Gruppe umfassend Antistatika, Antioxidantien, Antiblockmittel, Antifogmittel, antimikrobielle Wirkstoffe, Farbstoffe, Farbpigmente, Stabilisierungsmittel, vorzugsweise Hitze-Stabilisatoren, Prozess-Stabilisatoren, Prozesshilfsmittel, Flammschutzmittel, Nukleierungsmittel, Kristallisationsmittel, vorzugsweise Kristallkeimbildner, Gleitmittel, optische Aufheller, Flexibilisierungsmittel, Siegelmittel, Weichmacher, Silane, Abstandshalter, Füllstoffe, Peel-Additive, Wachse, Benetzungsmittel, oberflächenaktive Verbindungen, vorzugsweise Tenside, und Dispergiermittel enthalten.

Die (i) erste Schicht des mindestens dreischichtigen ersten Materials, die (ii) zweite Schicht des mindestens dreischichtigen ersten Materials und/oder die (iii) dritte Schicht des mindestens dreischichtigen ersten Materials können, jeweils unabhängig voneinander, 0,01-20 Gew.-%, vorzugsweise 0,1-10 Gew.-%, jeweils bezogen auf das Gesamtgewicht einer einzelnen Schicht, an wenigstens einem der vorstehend genannten Zusatzstoffe enthalten.

Neben der dritten Schicht des ersten Materials, welche orientiertes Polypropylen (z. B. (B)OPP) enthält oder daraus besteht, kann das Gehäuse ggfs. zusätzlich eine weitere Barriereschicht mit einer Barrierefunktion gegen die Permeation des flüchtigen Stoffs aufweisen.

Bevorzugt besteht die zusätzliche Barriereschicht aus Polyamid, Polyester, Aluminium, Ethylen-Vinylalkohol-Copolymer, Siliziumdioxid und/oder Polyvinylalkohol. Diese Materialien weisen gute Sperreigenschaften, insbesondere eine geringe Permeabilität für flüchtige Stoffe, insbesondere Duftstoffe oder insektizide Wirkstoffe, auf. Eine Ausgestaltung mit Polyvinylalkohol bietet den Vorteil, dass die zusätzliche Barriereschicht wasserlöslich ist und daher vereinfacht entsorgt werden kann. Insgesamt wird so ein vereinfachtes Recycling der Vorrichtung ermöglicht.

Das erste Material kann auch mehrere, vorzugsweise zwei, drei, vier oder fünf, zusätzliche Barriereschichten aufweisen. Jede einzelne der zusätzlichen Barriereschichten kann aus Polyamid, Polyester, Aluminium, Ethylen-Vinylalkohol-Copolymer, Siliziumdioxid und/oder Polyvinylalkohol bestehen. Beispielsweise kann vorgesehen sein, dass die zusätzlichen Barriereschichten unterschiedliche Permeabilitäten für unterschiedliche flüchtige Stoffe aufweisen. Auf diese Weise kann ein Gehäuse erhalten werden, welches eine Barrierefunktion für mehrere flüchtige Stoffe aufweist, beispielsweise für mehrere Stoffe einer Stoffmischung, z.B. einer Duftstoffmischung.

Bevorzugt ist die zusätzliche Barriereschicht zwischen zwei Schichten des ersten Materials angeordnet, vorzugsweise zwischen der ersten Schicht des ersten Materials und der zweiten Schicht des ersten Materials oder der zweiten Schicht des ersten Materials und der dritten Schicht des ersten Materials. Hierdurch kann eine kompakte Ausgestaltung des Gehäuses erhalten werden. Ferner kann die zusätzliche Barriereschicht durch die beiden Schichten des ersten Materials vor Umwelteinflüssen geschützt werden. Bei Verwendung einer zusätzlichen Barriereschicht aus Polyvinylalkohol kann diese durch die beiden Schichten des ersten Materials beispielsweise vor der Einwirkung von Wasser geschützt werden und somit eine unerwünschte Schädigung der zusätzlichen Barriereschicht verhindert werden. Optional kann zwischen der zusätzlichen Barriereschicht und den beiden Schichten des ersten Materials jeweils eine weitere zusätzliche Schicht angeordnet sein, beispielsweise eine Schutzschicht und/oder eine Versiegelungsschicht.

Alternativ ist es bevorzugt, wenn die zusätzliche Barriereschicht als Beschichtung einer Schicht des ersten Materials ausgebildet ist, insbesondere als Beschichtung auf einer Innenseite des Gehäuses, d.h. der dritten Schicht des ersten Materials.

In einer bevorzugten Ausführungsform enthält das erste Material exakt drei Schichten, nämlich (i) eine erste Schicht, welche Papier, Karton oder Pappe, insbesondere Karton, enthält oder daraus besteht, (ii) eine zweite Schicht, welche Polyethylen (PE) enthält oder daraus besteht und (iii) eine dritte Schicht, welche orientiertes Polypropylen (z.B. (B)OPP) enthält oder daraus besteht. Ein solches Aufbau entspricht der Anordnung "Papier, Karton oder Pappe als äußere Schicht - Polyethylen als Haftvermittler - orientiertes Polypropylen als innere Schicht".

Eine vorteilhafte Ausgestaltung der Erfindung sieht vor, dass das Gehäuse eine Standfläche aufweist, auf welcher das Gehäuse auf einem Untergrund stehen kann, wobei die Standfläche wasserabweisend ausgebildet ist. Beispielsweise kann das Gehäuse im Bereich der Standfläche eine wasserabweisende Beschichtung aufweisen. Die Beschichtung kann ein Nanomaterial umfassen.

Eine vorteilhafte Ausgestaltung der Erfindung sieht vor, dass der pflanzliche Faserstoff ein Papier oder ein Karton oder eine Pappe, beispielsweise Holzschliffpappe oder eine Zellstoffpappe, ist.

Eine vorteilhafte Ausgestaltung der Erfindung sieht vor, dass der pflanzliche Faserstoff Holzstoff, insbesondere Holzschliff, oder Zellstoff oder Baumwollfaser oder Grasfaser ist.

Eine vorteilhafte Ausgestaltung der Erfindung sieht vor, dass das mineralische Material Steinmehl oder Bentonit oder Silikagel ist.

Eine vorteilhafte Ausgestaltung der Erfindung sieht vor, dass die Einlage mehrere miteinander, insbesondere durch einen Klebstoff, verbundene Lagen des zweiten Materials aufweist, insbesondere mehrere Lagen Holzschliffpappe. Dadurch, dass die Einlage aus mehreren Lagen des pflanzlichen Faserstoffs gebildet wird, kann das Gewicht der Einlage und damit auch das Gewicht der gesamten Vorrichtung eingestellt werden. Es hat sich herausgestellt, dass die Benutzer derartige Vorrichtungen als wertiger ansehen, wenn die Vorrichtungen ein gewisses Gewicht aufweisen - also nicht zu leicht sind. Daher kann durch die Wahl der Anzahl an Lagen, beispielsweise vier oder fünf oder sechs oder sieben oder acht, ein vorgegebenes Gewicht der Vorrichtung eingestellt werden. Zudem ergibt sich durch das erhöhte Gewicht der Einlage der Vorteil, dass sich der flüchtige Stoff, insbesondere der Duftstoff bzw. der insektizide Wirkstoff, über eine größere Masse der Einlage verteilt. Hierdurch wird der flüchtige Stoff derart verdünnt, dass in der unmittelbaren Nähe des Gehäuses der Vorrichtung kein unangenehm starker Geruch wahrnehmbar ist. Auch dieser Umstand kann dazu beitragen, dass die Vorrichtung von dem Benutzer als wertiger empfunden wird. Alternativ zu dem Verbinden durch einen Klebstoff kann das Verbinden der Lagen durch einen oder mehrere Nägel, Klammern, Ösen, Bolzen oder Streifen erfolgen, beispielsweise einen Streifen aus Metall, Holz, einem Polymer, Glas oder Stein.

Falls die Einlage mehrere Lagen Holzschliffpappe aufweist ist es vorteilhaft, wenn die Holzschliffpappe eine Schicht Holzschliff umfasst, die zwischen zwei Deckschichten angeordnet ist. Bevorzugt umfassen die Deckschichten jeweils Zellstoff oder Papier, besonders bevorzugt Zellstoff und Papier. Die Holzschliffpappe hat bevorzugt eine Dicke im Bereich von 0,5 mm bis 2,5 mm, beispielsweise 0,8 mm, 0,9 mm, 1,2 mm, 1,4 mm, 1,6 mm, 1,8 mm, 2,0 mm oder 2,4 mm.

Gemäß einer vorteilhaften Ausgestaltung ist vorgesehen, dass die Lagen jeweils ein Flächengewicht im Bereich von 350 g/m² bis 950 g/m², bevorzugt von 450 g/m² bis 600 g/m² oder von 600 g/m² bis 800 g/m², aufweisen. Lagen mit einem derartigen Flächengewicht haben sich als besonders gut zur Einstellung des Gewichts der Einlage erwiesen.

Alternativ ist es vorteilhaft, wenn die Lagen jeweils eine Dicke im Bereich von 0,2 mm bis 2,5 mm und ein Flächengewicht im Bereich von 350 g/m² bis 950 g/m² aufweisen,
bevorzugt
- eine Dicke im Bereich von 0,8 mm bis 1,0 mm und ein Flächengewicht im Bereich von 350 g/m² bis 400 g/m² oder
- eine Dicke im Bereich von 1,1 mm bis 1,3 mm und ein Flächengewicht im Bereich von 450 g/m² bis 550 g/m² oder
- eine Dicke im Bereich von 1,3 mm bis 1,5 mm und ein Flächengewicht im Bereich von 490 g/m² bis 570 g/m² oder
- eine Dicke im Bereich von 1,5 mm bis 1,7 mm und ein Flächengewicht im Bereich von 580 g/m² bis 660 g/m² oder
- eine Dicke im Bereich von 1,7 mm bis 1,9 mm und ein Flächengewicht im Bereich von 630 g/m² bis 750 g/m² oder
- eine Dicke im Bereich von 1,9 mm bis 2,1 mm und ein Flächengewicht im Bereich von 700 g/m² bis 850 g/m² oder
- eine Dicke im Bereich von 2,3 mm bis 2,5 mm und ein Flächengewicht im Bereich von 800 g/m² bis 1000 g/m².

Lagen mit einer derartigen Kombination aus Dicke und Flächengewicht haben sich als besonders gut zur Einstellung des Gewichts der Einlage erwiesen.

Gemäß einer vorteilhaften Ausgestaltung der Erfindung ist vorgesehen, dass der Klebstoff biologisch abbaubar ist. Durch die Verwendung eines biologisch abbaubaren Klebstoffs kann eine Einlage bereitgestellt werden, die nochmals erleichtert entsorgt, insbesondere recycelt bzw. industriell kompostiert werden kann. Besonders bevorzugt entspricht der Klebstoff der europäischen Norm EN 13432 und ist in ihrem Sinne "vollständig biologisch abbaubar". Alternativ kann ein Klebstoff verwendet werden, der nicht biologisch abbaubar ist.

Eine vorteilhafte Ausgestaltung der Erfindung sieht vor, dass die Einlage mindestens eine erste Lage aufweist, die insbesondere vollflächig ausgebildet ist, und eine mindestes eine zweite Lage aufweist, die mindestens eine Ausnehmung, insbesondere Ausstanzung aufweist. Die Ausstanzung kann eine Aufnahme für das flüchtige Material bilden. Insofern ist es möglich, dass flüchtige Material, beispielsweise in flüssiger Form, in die durch die Ausnehmung gebildete Aufnahme zu geben. Das flüchtige Material kann ausgehend von der Ausnehmung in die Einlage einziehen. Die mindestens eine Ausnehmung kann länglich ausgebildet sein, beispielsweise als Rille.

In diesem Zusammenhang hat sich eine Ausgestaltung als vorteilhaft erwiesen, bei welcher die zweite Lage eine äußere Oberfläche der Einlage bildet.

Eine vorteilhafte Ausgestaltung sieht vor, dass die Einlage mehrere zweite Lagen aufweist, wobei die Ausnehmungen der mehreren zweiten Lagen deckungsgleich übereinander angeordnet sind.

Eine vorteilhafte Ausgestaltung der Erfindung sieht vor, dass auf einer Außenseite des Gehäuses ein Zeit-Temperatur-Indikator angeordnet ist. Durch den Zeit-Temperatur-Indikator kann das Ende der Verwendbarkeit der Vorrichtung angezeigt werden. Hierdurch kann einem Benutzer der Vorrichtung angezeigt werden, ob die Vorrichtung noch zu verwenden ist oder bereits entsorgt werden bzw. einem Recyclingprozess zugeführt werden kann. Bevorzugt umfasst der Zeit-Temperatur-Indikator wärme- und UV-lichtempfindliche Pigmente. Diese Pigmente können durch einen Hersteller der Vorrichtung am Ende des Herstellungsprozesses durch Einstrahlung von UV-Licht aktiviert werden, wobei sich die dabei verwendete UV-Dosis nach der Haltbarkeitsdauer richtet. Besonders bevorzugt sind die wärme- und UV-lichtempfindliches Pigmente durch eine UV-Licht-undurchlässige Folie abgedeckt, sodass ein späteres Manipulieren durch UV-Licht nicht möglich ist.

Eine vorteilhafte Ausgestaltung der Erfindung sieht vor, dass das Gehäuse ein erstes Gehäuseteil und ein zweites Gehäuseteil aufweist, wobei das erste Gehäuseteil und/oder das zweite Gehäuseteil eine Aufnahme für die Einlage umfasst, wobei das erste Gehäuseteil eine oder mehrere Öffnungen, insbesondere Durchgangsöffnungen, aufweist, durch welche der flüchtige Stoff in die Umgebungsluft abgegeben werden kann, und das zweite Gehäuseteil aus einer Schließstellung, in welcher es die Öffnungen, insbesondere Durchgangsöffnungen, des ersten Gehäuseteils verdeckt, in eine Offenstellung verschiebbar ist, in welcher die Öffnungen, insbesondere Durchgangsöffnungen, des ersten Gehäuseteils zur Abgabe des flüchtigen Stoffs freigegeben sind. Bei einer derartigen Ausgestaltung kann die Abgabe des flüchtigen Stoffs durch Verschieben der beiden Gehäuseteile gegeneinander aktiviert werden. In der Schließstellung verdeckt das zweite, insbesondere äußere, Gehäuseteil die Durchgangsöffnungen in dem ersten, insbesondere inneren, Gehäuseteil. Da das erste Material des inneren und äußeren Gehäuseteils ein Austreten des flüchtigen Stoffs begrenzen, insbesondere verhindern, bleibt der Partialdruck des flüchtigen Stoffs innerhalb des Gehäuses konstant auf einem Niveau, das einen Austritt des flüchtigen Stoffs aus der Einlage verhindert. Bei Aktivierung der Vorrichtung durch das Verschieben der Gehäuseteile werden die eine oder mehreren Durchgangsöffnungen freigegeben. Folglich kann die Umgebungsluft durch die Durchgangsöffnungen zirkulieren und der flüchtige Stoff in die Umgebungsluft abgegeben werden.

Das erste und das zweite Gehäuseteil haben bevorzugt einen Grundriss von identischer Form, beispielsweise rund, dreieckig, viereckig, insbesondere quadratisch, fünfeckig, sechseckig oder achteckig.

Bevorzugt ist an dem ersten Gehäuseteil und/oder dem zweiten Gehäuseteil ein Rastelement angeordnet, dass ein vollständiges Trennen der beiden Gehäuseteile voneinander verhindert. Hierdurch kann ein unerwünschtes Freilegen der Einlage, insbesondere durch Kinder, verhindert werden. Insofern kann durch eine solche Ausgestaltung die Kindersicherheit erhöht werden.

Eine alternative, vorteilhafte Ausgestaltung der Erfindung sieht vor, dass das Gehäuse ein erstes Gehäuseteil, ein zweites Gehäuseteil und ein drittes Gehäuseteil aufweist, wobei das erste Gehäuseteil und/oder das zweite Gehäuseteil eine Aufnahme für die Einlage umfasst, wobei das dritte Gehäuseteil eine oder mehrere Durchgangsöffnungen aufweist, durch welche der flüchtige Stoff in die Umgebungsluft abgegeben werden kann, wobei das erste Gehäuseteil und/oder das zweite Gehäuseteil aus einer Schließstellung, in welcher das erste Gehäuseteil und/oder das zweite Gehäuseteil die Durchgangsöffnungen des dritten Gehäuseteils verdeckt, in eine Offenstellung verschiebbar ist, in welcher die Durchgangsöffnungen des dritten Gehäuseteils zur Abgabe des flüchtigen Stoffs freigegeben sind. Bei einer derartigen Ausgestaltung kann die Abgabe des flüchtigen Stoffs durch Verschieben des ersten und/oder des zweiten Gehäuseteils gegeneinander dem dritten Gehäuseteil werden. In der Schließstellung verdeckt das erste und/oder zweite Gehäuseteil die Durchgangsöffnungen in dem dritten Gehäuseteil, welches in der Schließstellung bevorzugt innerhalb des ersten und/oder zweiten Gehäuseteils angeordnet ist. Da das erste Material des Gehäuses ein Austreten des flüchtigen Stoffs begrenzen, insbesondere verhindern, bleibt der Partialdruck des flüchtigen Stoffs innerhalb des Gehäuses konstant auf einem Niveau, das einen Austritt des flüchtigen Stoffs aus der Einlage verhindert. Bei Aktivierung der Vorrichtung durch das Verschieben des ersten und/oder zweiten Gehäuseteils gegenüber dem dritten Gehäuseteil werden die eine oder mehreren Durchgangsöffnungen freigegeben. Folglich kann die Umgebungsluft durch die Durchgangsöffnungen zirkulieren und der flüchtige Stoff in die Umgebungsluft abgegeben werden.

Bei einer derartigen Ausgestaltung können das erste Gehäuseteil und das zweite Gehäuseteil identisch ausgebildet sein. Das dritte Gehäuseteil kann die Form eines Rohrs aufweisen, wobei ein Grundriss des Rohrs von identischer Form ist wie ein Grundriss des ersten und/oder zweiten Gehäuseteils, insbesondere rund, dreieckig, viereckig, insbesondere quadratisch, fünfeckig, sechseckig oder achteckig.

Bevorzugt ist an dem dritten Gehäuseteil ein erstes Rastelement angeordnet, welchem mit einem weiteren ersten Rastelement an dem ersten Gehäuseteil zusammenwirkt und an dem dritten Gehäuseteil ist ein zweites Rastelement angeordnet, welches mit einem weiteren zweiten Rastelement an dem zweiten Gehäuseteil zusammenwirkt. Durch diese Rastelemente kann ein vollständiges Trennen des ersten und zweiten Gehäuseteils von dem dritten Gehäuseteil unterbunden werden. Hierdurch kann ein unerwünschtes Freilegen der Einlage, insbesondere durch Kinder, verhindert werden. Insofern kann durch eine solche Ausgestaltung die Kindersicherheit erhöht werden.

Gemäß einer vorteilhaften Ausgestaltung ist ein Spalt zwischen dem ersten Gehäuseteil und dem zweiten Gehäuseteil in der Schließstellung durch ein entfernbares Barriereelement abgedeckt, welches ein Barrierematerial mit einer Barrierefunktion gegen die Permeation des flüchtigen Stoffs aufweist oder daraus ausgebildet ist. Das Barrierematerial kann beispielsweise Polyamid, Polyester, Aluminium oder Polyvinylalkohol sein. Bevorzugt ist das entfernbare Barriereelement als Banderole ausgebildet. Alternativ kann vorgesehen sein, dass das entfernbare Barriereelement als Release-Folie ausgebildet ist.

Bei einer Vorrichtung gemäß einem der vorstehend beschriebenen Ausgestaltungen mit einem ersten und einem zweiten und ggf. einem dritten Gehäuseteil ist bevorzugt vorgesehen, dass das zweite Gehäuseteil ausgehend von der Schließstellung derart in Richtung der Offenstellung bewegbar ist, dass dabei Umgebungsluft in das Gehäuse gesaugt wird und/oder dass das zweite Gehäuseteil ausgehend von der Offenstellung derart in Richtung der Schließstellung bewegbar ist, dass dabei mit dem flüchtigen Stoff angereicherte Luft aus dem Gehäuse gedrückt wird. Durch ein aufeinanderfolgendes Öffnen und Schließen des Gehäuses, d.h. ein aufeinanderfolgendes Überführen von der Schließstellung in die Offenstellung und dann wieder in Richtung der Schließstellung, kann eine kurzfristige stärkere Abgabe des flüchtigen Stoffs aus der Vorrichtung erreicht werden, beispielsweise eine kurzfristige, stärkere Duftabgabe an die Umgebungsluft.

Eine alternative vorteilhafte Ausgestaltung sieht vor, dass das Gehäuse eine Aufnahme für die Einlage und ein oder mehrere Durchgangsöffnungen aufweist, durch welche der flüchtige Stoff in die Umgebungsluft abgegeben werden kann, wobei die Durchgangsöffnungen mit einem entfernbaren Barriereelement, beispielsweise einer Folie, versehen sind. Das Barriereelement kann beispielsweise als Release-Folie ausgebildet sein. Alternativ kann vorgesehen sein, dass das entfernbare Barriereelement ein entfernbares Gehäuseelement aus dem ersten Material des Gehäuses ist. Beispielsweise kann das entfernbares Gehäuseelement über eine Perforation mit dem Rest des Gehäuses verbunden sein. Beim Entfernen des Gehäuseelements kann diese Perforation aufgetrennt werden.

Weitere Einzelheiten, Merkmale und Vorteile der Erfindung ergeben sich aus den Zeichnungen, sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den Erfindungsgedanken nicht einschränken.

### Kurze Beschreibung der Figuren

Die **Fig. 1** zeigt eine perspektivische Darstellung einer Vorrichtung zur Abgabe eines flüchtigen Stoffs an die Umgebungsluft gemäß einem ersten Ausführungsbeispiel in einer Schließstellung.
Die **Fig. 2** zeigt die Vorrichtung nach Fig. 1 beim Lösen des Dampfsperrelements.
Die **Fig. 3** zeigt die Vorrichtung nach Fig. 1 in einer Offenstellung.
Die **Fig. 4** zeigt eine perspektivische Darstellung einer Vorrichtung zur Abgabe eines flüchtigen Stoffs an die Umgebungsluft gemäß einem zweiten Ausführungsbeispiel in einer Schließstellung.
Die **Fig. 5** zeigt die Vorrichtung nach Fig. 3 in einer Offenstellung.
Die **Fig. 6** zeigt eine perspektivische Darstellung einer Vorrichtung zur Abgabe eines flüchtigen Stoffs an die Umgebungsluft gemäß einem dritten Ausführungsbeispiel in einer Offenstellung.
Die **Fig. 7** zeigt ein inneres Gehäuseteil eines Gehäuses der Vorrichtung nach Fig. 6.
Die **Fig. 8** zeigt die Vorrichtung nach Fig. 6 in einer Schließstellung.
Die **Fig. 9a** zeigt eine Einlage der Vorrichtung nach Fig. 1 in einer Draufsicht.
Die **Fig. 9b** zeigt eine Einlage der Vorrichtung nach Fig. 1 in einer Seitenansicht.
Die **Fig. 10a** zeigt eine Einlage der Vorrichtung nach Fig. 4 in einer Draufsicht.
Die **Fig. 10b** zeigt eine Einlage der Vorrichtung nach Fig. 4 in einer Seitenansicht.
Die **Fig. 11** zeigt eine alternative Einlage der Vorrichtungen nach Fig. 1, 4 und 6 in einer perspektivischen Darstellung.

### Ausführungsformen der Erfindung

In den verschiedenen Figuren sind gleiche Teile stets mit den gleichen Bezugszeichen versehen und werden daher in der Regel auch jeweils nur einmal benannt bzw. erwähnt.

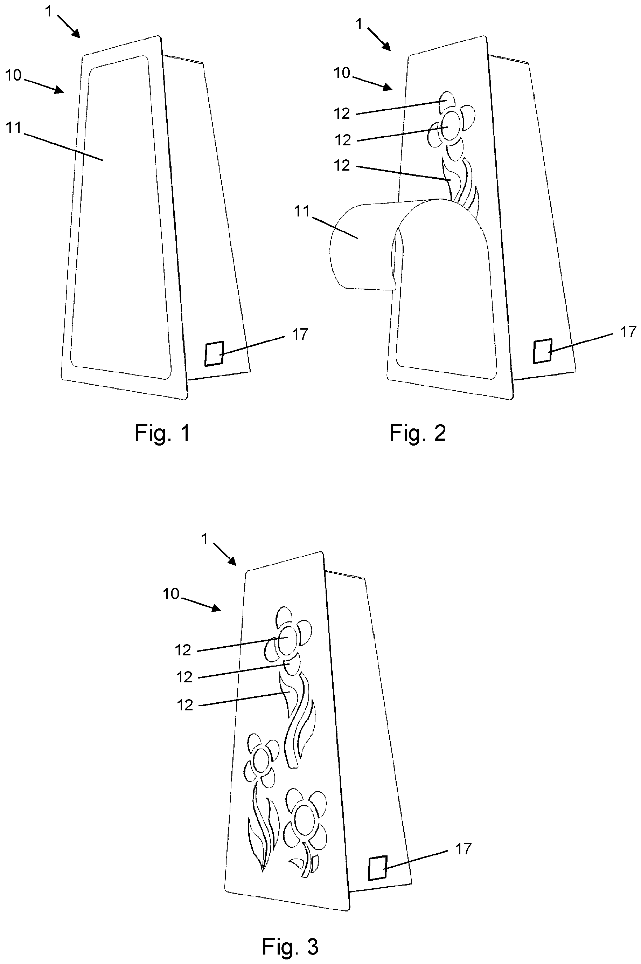

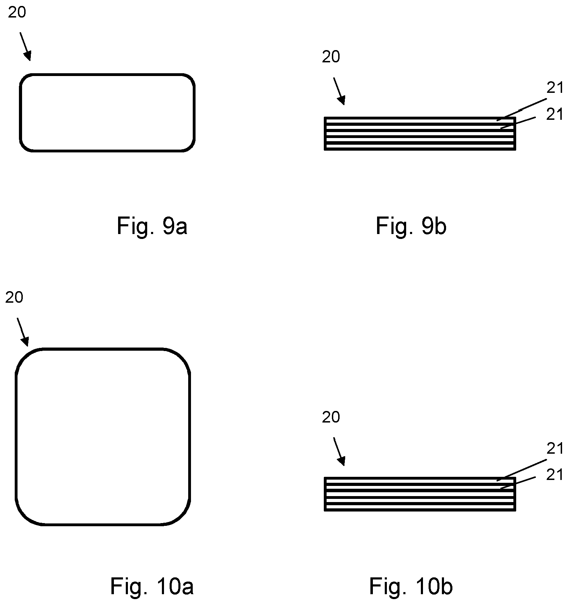

Die Darstellung in **Fig. 1** zeigt eine perspektivische Darstellung einer Vorrichtung 1 zur Abgabe eines flüchtigen Stoffs, beispielsweise in Form eines Duftstoffs oder insektiziden Wirkstoffs, an die Umgebungsluft gemäß einem ersten Ausführungsbeispiel der Erfindung. Die Vorrichtung 1 umfasst ein Gehäuse 10 aus einem dreischichtigen ersten Material sowie ferner eine Barriereschicht mit einer Barrierefunktion gegen die Permeation des flüchtigen Stoffs auf sowie eine in Fig. 9a und 9b dargestellte Einlage 20, die innerhalb des Gehäuses 10 angeordnet ist. Die Einlage 20 besteht aus einem zweiten Material, welches ein pflanzlicher Faserstoff, insbesondere ein Papier, ein Karton oder eine Pappe ist, wobei das zweite Material mit dem flüchtigen Stoff versehen, insbesondere imprägniert, ist.

Das Gehäuse 10 umfasst eine Aufnahme für die Einlage und ein oder mehrere Durchgangsöffnungen 12, durch welche der flüchtige Stoff in die Umgebungsluft abgegeben werden kann, wobei die Durchgangsöffnungen 12 in einer Schließstellung des Gehäuses 10 mit einem entfernbaren Barriereelement 11, beispielsweise einer Folie, versehen sind, vgl. Fig. 1. Alternativ kann vorgesehen sein, dass das entfernbare Barriereelement 11 ein entfernbares Gehäuseelement aus dem ersten Material des Gehäuses 10 ist. Beispielsweise kann das entfernbares Gehäuseelement über eine Perforation mit dem Rest des Gehäuses 10 verbunden sein. Beim Entfernen des Gehäuseelements 11 kann diese Perforation aufgetrennt werden.

Die Darstellung in **Fig. 2** zeigt beispielhaft das Entfernen dieses Barriereelements 11 von den Durchgangsöffnungen 12.

In **Fig. 3** ist das Gehäuse 10 in einer Offenstellung gezeigt, in welcher die Durchgangsöffnungen freiliegen, so dass Umgebungsluft durch diese zirkulieren kann und der flüchtige Stoff, beispielsweise Duftstoff, der Einlage 20 aus dem inneren des Gehäuses 10 austreten kann. Das Barriereelement 11 ist gemäß Fig. 3 vollständig von dem Gehäuse 10 entfernt.

Das dreischichtige erste Material des Gehäuses 10 der Vorrichtung 1 weist eine erste, nach Schicht aus Papier, Karton oder Pappe, eine zweite Schicht aus Polyethylen und eine dritte Schicht aus orientiertem Polypropylen oder biaxial orientiertem Polypropylen auf. Es umfasst ferner eine Barriereschicht bzw. ein Barriereelement 11, beispielsweise bestehend aus Polyamid, Polyester, Aluminium oder Polyvinylalkohol. Diese Barriereschicht kann in Kontakt zu einer Lage des dreischichten ersten Materials, insbesondere des Papiers, des Kartons oder der Pappe angeordnet sein. Ferner kann vorgesehen sein, dass die Barriereschicht aus dem dreischichtigen Material aus Papier, Karton oder Pappe, Polyethylen und orientiertem Polypropylen oder biaxial orientiertem Polypropylen gebildet ist.

An der Außenseite des Gehäuses 10 ist ferner ein Zeit-Temperatur-Indikator 17 angeordnet, über welchen die Haltbarkeit des flüchtigen Stoffs angezeigt werden kann.

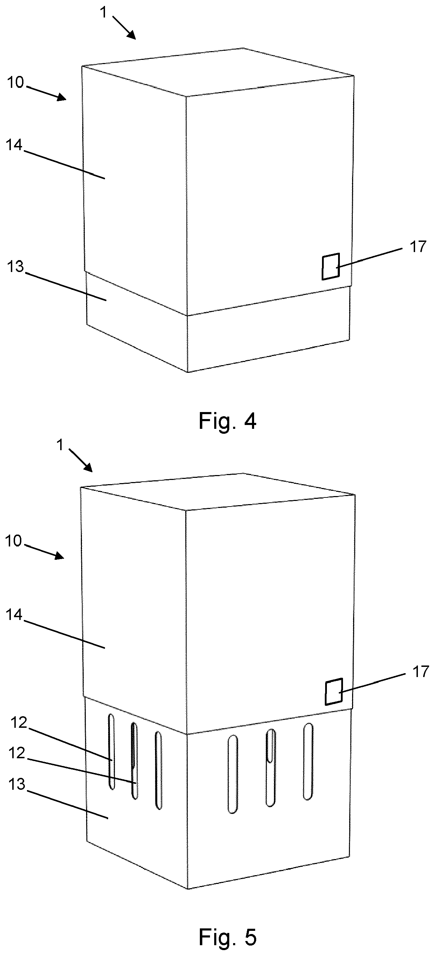

In **Fig. 4 und 5** ist ein zweites Ausführungsbeispiel einer erfindungsgemäßen Vorrichtung 1 zur Abgabe eines flüchtigen Stoffs an die Umgebungsluft dargestellt. Auch die Vorrichtung 1 gemäß dem zweiten Ausführungsbeispiel umfasst ein Gehäuse 10 aus einem ersten dreischichtigen Material auf, welches aus einer ersten Schicht aus Papier, Karton oder Pappe, aus einer zweiten Schicht aus Polyethylen sowie einer dritten Schicht aus orientiertem Polypropylen oder biaxial orientiertem Polypropylen gebildet ist. Weiterhin weist es eine zusätzliche Barriereschicht mit einer Barrierefunktion gegen die Permeation des flüchtigen Stoffs auf sowie eine in Fig. 10a und 10b dargestellte Einlage 20, die innerhalb des Gehäuses 10 angeordnet ist. Die Einlage 20 besteht aus einem zweiten Material, welches ein pflanzlicher Faserstoff, insbesondere ein Papier, ein Karton oder eine Pappe, ist, wobei das zweite Material mit dem flüchtigen Stoff versehen, insbesondere imprägniert, ist.

Das zu dem ersten Material gemäß dem ersten Ausführungsbeispiel gesagte trifft auch auf das zweite Ausführungsbeispiel zu. Daher wird auf die Beschreibung zu Fig. 1-3 verwiesen.

Im Unterschied zu dem ersten Ausführungsbeispiel umfasst das Gehäuse 10 gemäß dem zweiten Ausführungsbeispiel ein erstes Gehäuseteil 13 mit einer Aufnahme für die Einlage, wobei das erste Gehäuseteil 13 eine oder mehrere Durchgangsöffnungen 12 aufweist, durch welche der flüchtige Stoff in die Umgebungsluft abgegeben werden kann, und ein zweites Gehäuseteil 14, welches aus einer in **Fig. 4** gezeigten Schließstellung, in welcher es die Durchgangsöffnungen 12 des ersten Gehäuseteils verdeckt, in eine in **Fig. 5** gezeigte Offenstellung verschoben werden kann, in welcher die Durchgangsöffnungen des ersten Gehäuseteils zur Abgabe des flüchtigen Stoffs freigegeben sind.

Auch das Gehäuse 10 gemäß dem zweiten Ausführungsbeispiel weist einen Zeit-Temperatur-Indikator 17 auf, über welchen die Haltbarkeit des Duftstoffs bzw. des insektiziden Wirkstoffs angezeigt werden kann.

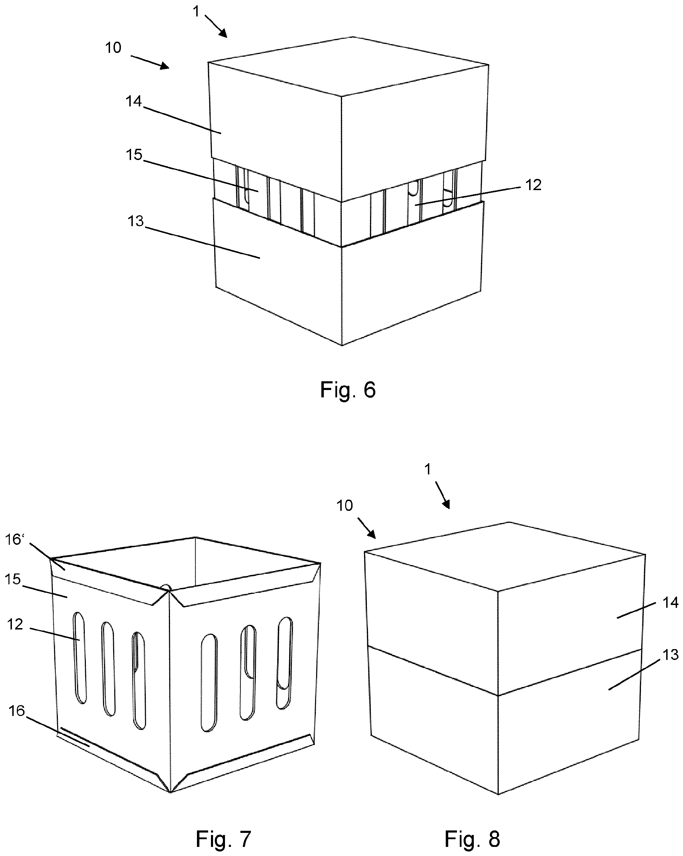

In **Fig. 6** ist ein drittes Ausführungsbeispiel einer erfindungsgemäßen Vorrichtung 1 zur Abgabe eines flüchtigen Stoffs an die Umgebungsluft dargestellt. Auch die Vorrichtung 1 gemäß dem dritten Ausführungsbeispiel umfasst ein Gehäuse 10 aus einem ersten dreischichtigen Material auf, welches aus einer ersten Schicht aus Papier, Karton oder Pappe, aus einer zweiten Schicht aus Polyethylen sowie einer dritten Schicht aus orientiertem Polypropylen oder biaxial orientiertem Polypropylen gebildet ist sowie eine zusätzliche Barriereschicht mit einer Barrierefunktion gegen die Permeation des flüchtigen Stoffs sowie eine in Fig. 10a und 10b dargestellte Einlage 20, die innerhalb des Gehäuses 10 angeordnet ist. Die Einlage 20 besteht aus einem zweiten Material, welches ein pflanzlicher Faserstoff, insbesondere ein Papier, ein Karton oder eine Pappe, ist, wobei das zweite Material mit dem flüchtigen Stoff versehen, insbesondere imprägniert, ist.

Das zu dem ersten Material gemäß dem ersten Ausführungsbeispiel gesagte trifft auch auf das zweite Ausführungsbeispiel zu. Daher wird auf die Beschreibung zu Fig. 1-3 verwiesen.

Das Gehäuse 10 der Vorrichtung 1 gemäß dem dritten Ausführungsbeispiel umfasst ein erstes Gehäuseteil 13, ein zweites Gehäuseteil 14 und ein drittes Gehäuseteil 15. Das erste und das zweite Gehäuseteil 13, 14 sind bei dem Ausführungsbeispiel identisch ausgebildet. Sowohl das erste als auch das zweite Gehäuseteil 13, 14 weisen eine Aufnahme für die Einlage 20 auf, es ist jedoch nur eines der beiden Gehäuseteile 13, 14 mit der Einlage 20 bestückt.

Das dritte Gehäuseteil 15 ist nach Art eines Rohrs bzw. nach Art einer Hülse ausgebildet und umfasst mehrere Durchgangsöffnungen 12, durch welche der flüchtige Stoff in die Umgebungsluft abgegeben werden kann, sofern diese durch das erste und/oder zweite Gehäuseteil 13, 14 freigegeben sind. Denn in der in **Fig. 8** gezeigten Schließstellung verdecken das erste Gehäuseteil 13 und das zweite Gehäuseteil 14 die Durchgangsöffnungen 12 des dritten Gehäuseteils 15. Das erste und zweite Gehäuseteil 13, 14 sind aus dieser Schließstellung in eine Offenstellung verschiebbar, welche in Fig. 6 gezeigt ist und in welcher die Durchgangsöffnungen 12 des dritten Gehäuseteils 15 zur Abgabe des flüchtigen Stoffs freigegeben sind.

Das Gehäuse 10 kann zusätzlich ein entfernbares Barriereelement aufweisen, welches einen Spalt zwischen dem ersten Gehäuseteil 13 und dem zweiten Gehäuseteil 14 in der Schließstellung abdeckt, beispielsweise ein als Banderole ausgebildetes Barriereelement. Dieses Barrierelement weist ein Barrierematerial mit einer Barrierefunktion gegen die Permeation des flüchtigen Stoffs auf oder ist aus einem solchen ausgebildet.

Das dritte Gehäuseteil 15 des Gehäuses 10 ist in **Fig. 7** dargestellt. Dieser Darstellung kann entnommen werden, dass an dem dritten Gehäuseteil mehrere erste Rastelemente 16 angeordnet sind, welchem mit weiteren ersten Rastelementen an dem ersten Gehäuseteil 13 zusammenwirken können. Ferner umfasst das dritte Gehäuseteil 15 mehrere zweite Rastelemente 16`, die mit weiteren zweiten Rastelementen an dem zweiten Gehäuseteil 14 zusammenwirken können. Die ersten Rastelemente 16 sind dabei auf einer ersten Seite des dritten Gehäuseteils 15 angeordnet, die einer zweiten Seite des dritten Gehäuseteils gegenüberliegt, auf welcher die zweiten Rastelemente 16' angeordnet sind. Insofern sind bei zusammengesetztem Gehäuse die ersten Rastelemente 16 dem ersten Gehäuseteil 13 und die zweiten Rastelemente 16' dem zweiten Gehäuseteil 14 zugewandt. Die ersten und zweiten Rastelemente 16, 16' sind als umgefalzte Abschnitte des dritten Gehäuseteils 13 ausgestaltet.

Ein Gehäuse 10 in der Schließstellung mit einem ersten Gehäuseteil 13 und einem zweiten Gehäuseteil 14 ist in **Fig. 8** gezeigt.

**Fig. 9a und 9b** zeigen die Einlage 20 des ersten Ausführungsbeispiels. Diese Einlage 20 weist einen rechteckigen Querschnitt mit abgerundeten Ecken auf. Die Einlage 20 besteht aus mehreren, hier fünf, miteinander durch einen Klebstoff verbundenen Lagen 21 des pflanzlichen Faserstoffs, beispielsweise Holzschliffpappe oder Zellstoffpappe. Der Klebstoff ist bevorzugt biologisch abbaubar. Die Lagen weisen jeweils ein Flächengewicht im Bereich von 350 g/m² bis 950 g/m², bevorzugt von 450 g/m² bis 600 g/m² oder von 600 g/m² bis 800 g/m², auf. Alternativ kann vorgesehen sein, dass die Lagen jeweils eine Dicke im Bereich von 0,8 mm bis 2,5 mm und ein Flächengewicht im Bereich von 350 g/m² bis 950 g/m² aufweisen.

**Fig. 10a und 10b** zeigen die Einlage 20 des zweiten und dritten Ausführungsbeispiels. Diese Einlage 20 weist einen quadratischen Querschnitt mit abgerundeten Ecken auf. Die Einlage 20 besteht aus mehreren, hier fünf, miteinander durch einen Klebstoff verbundenen Lagen 21 des pflanzlichen Faserstoffs, beispielsweise Holzschliffpappe oder Zellstoffpappe. Der Klebstoff ist bevorzugt biologisch abbaubar. Die Lagen weisen jeweils ein Flächengewicht im Bereich von 350 g/m² bis 950 g/m², bevorzugt von 450 g/m² bis 600 g/m² oder von 600 g/m² bis 800 g/m², auf. Alternativ kann vorgesehen sein, dass die Lagen jeweils eine Dicke im Bereich von 0,8 mm bis 2,5 mm und ein Flächengewicht im Bereich von 350 g/m² bis 950 g/m² aufweisen.

Die in Fig. 6a, 6b, 7a und 7b gezeigten Ausführungsbeispiele der Einlagen 20 sind lediglich beispielhaft und können abweichend davon eine andere Form aufweisen, z.B. fünfeckig, sechseckig oder achteckig sein, und/oder spitze, nicht-abgerundete Ecken aufweisen.

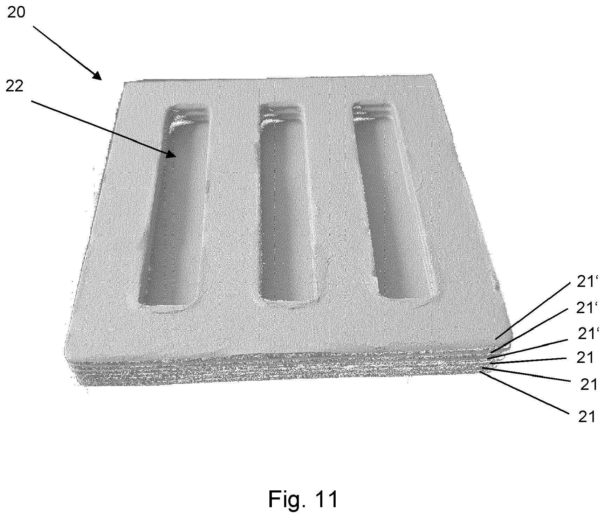

**Fig. 11** zeigt eine Einlage 20, die alternativ bei den vorstehend beschriebenen Ausführungsbeispielen Anwendung finden kann. Die Einlage 20 besteht aus mehreren, hier sechs, miteinander durch einen Klebstoff verbundenen Lagen 21 des pflanzlichen Faserstoffs, beispielsweise Holzschliffpappe oder Zellstoffpappe. Die Einlage 20 weist mehrere, hier drei erste Lagen 21 auf, die vollflächig ausgebildet sind. Ferner weist die Einlage 20 mehrere, hier drei zweite Lagen 21' auf, die mehrere, hier drei Ausnehmungen, insbesondere Ausstanzungen, umfassen. Eine der zweiten Lagen 21' bildet dabei eine äußere Oberfläche der Einlage 20. Die Ausnehmungen 22 der zweiten Lagen 21' sind deckungsgleich übereinander angeordnet und bilden eine Aufnahme für flüchtiges Material.

Die vorstehend beschriebenen Einlagen 20 können alternativ aus einem der folgenden Materialien bestehen oder eines oder mehrere der genannten Materialien aufweisen: ein anderer pflanzlicher Faserstoff oder Saccharose oder Stärke oder ein mineralisches Material. Der pflanzliche Faserstoff kann Holzstoff, insbesondere Holzschliff, oder Zellstoff oder Baumwollfaser oder Grasfaser sein. Das mineralische Material kann Steinmehl oder Bentonit oder Silikagel sein.

### Bezugszeichenliste

- 1: Vorrichtung zur Abgabe eines Duftstoffs oder insektiziden Wirkstoffs an die Umgebungsluft
- 10: Gehäuse
- 11: Barriereelement
- 12: Durchgangsöffnung
- 13: erstes Gehäuseteil
- 14: zweites Gehäuseteil
- 15: drittes Gehäuseteil
- 16, 16': Rastelement
- 17: Zeit-Temperatur-Indikator
- 20: Einlage
- 21, 21': Lage
- 22: Ausnehmung

## Patentansprüche

1. Vorrichtung (1) zur Abgabe eines flüchtigen Stoffs, insbesondere eines Duftstoffs oder eines insektiziden Wirkstoffs, an die Umgebungsluft, mit
**a)** einem Gehäuse (10), welches
- ein mindestens dreischichtiges erstes Material enthält und wobei
(i) eine erste Schicht des mindestens dreischichtigen ersten Materials ein Papier oder ein Karton oder eine Pappe enthält oder daraus besteht,
(ii) eine zweite Schicht des mindestens dreischichtigen ersten Materials Polyethylen enthält oder daraus besteht, und
(iii) eine dritte Schicht des mindestens dreischichtigen ersten Materials orientiertes Polypropylen enthält oder daraus besteht und diese dritte Schicht eine Barrierefunktion gegen die Permeation des flüchtigen Stoffs aufweist, und
- ggfs. wenigstens eine zusätzliche Barriereschicht mit einer Barrierefunktion gegen die Permeation des flüchtigen Stoffs aufweist,
und
**b)** einer innerhalb des Gehäuses (10) angeordneten Einlage (20) aus einem zweiten Material, wobei das zweite Material mit dem flüchtigen Stoff versehen, insbesondere imprägniert, ist, wobei das zweite Material eines der folgenden Materialien ist oder eines oder mehrere der genannten Materialien aufweist:
- ein pflanzlicher Faserstoff oder
- Saccharose oder Stärke oder
- ein mineralisches Material.

2. Vorrichtung nach Anspruch 1, **dadurch gekennzeichnet, dass** das Polyethylen Low-Density Polyethylen (LDPE / PE-LD), Linear-Low-Density Polyethylen (LLDPE / PE-LLD) und/oder High-Density Polyethylen (HDPE / PE-HD) umfasst oder ist.

3. Vorrichtung nach einem der vorhergehenden Ansprüche, **dadurch gekennzeichnet, dass** das orientierte Polypropylen OPP ("oriented polypropylene") oder BOPP ("biaxially oriented polypropylene") umfasst oder ist.

4. Vorrichtung nach einem der vorhergehenden Ansprüche, **dadurch gekennzeichnet, dass** die zusätzliche Barriereschicht aus Polyamid, Polyester, Aluminium und/oder Polyvinylalkohol besteht.

5. Vorrichtung nach einem der vorhergehenden Ansprüche, **dadurch gekennzeichnet, dass** (i) die erste Schicht und/oder (ii) die zweite Schicht und/oder (iii) die dritte Schicht zumindest teilweise eine Acrylbeschichtung aufweist/aufweisen.

6. Vorrichtung nach einem der vorhergehenden Ansprüche, **dadurch gekennzeichnet, dass** (iii) die dritte Schicht des ersten Materials, welche orientiertes Polypropylen enthält oder daraus besteht, wasserabweisend und/oder transparent ist.

7. Vorrichtung nach einem der vorhergehenden Ansprüche, **dadurch gekennzeichnet, dass** das erste Material
(i) eine erste Schicht aus Papier, Karton oder Pappe aufweist;
(ii) eine zweite Schicht aus Polyethylen aufweist,
(iii) eine dritte Schicht aus orientiertem Polypropylen aufweist, wobei diese Schicht eine Barrierefunktion gegen die Permeation des flüchtigen Stoffs aufweist, und
ggfs. wenigstens eine zusätzliche Barriereschicht mit einer Barrierefunktion gegen die Permeation des flüchtigen Stoffs aufweist,

8. Vorrichtung nach einem der vorhergehenden Ansprüche, **dadurch gekennzeichnet, dass** das Gehäuse (10) eine Standfläche aufweist, auf welcher das Gehäuse (10) auf einem Untergrund stehen kann, wobei die Standfläche wasserabweisend ausgebildet ist.

9. Vorrichtung nach einem der vorhergehenden Ansprüche, **dadurch gekennzeichnet, dass** der pflanzliche Faserstoff ein Papier oder ein Karton oder eine Pappe, beispielsweise Holzschliffpappe oder eine Zellstoffpappe, ist.

10. Vorrichtung nach einem der vorhergehenden Ansprüche, **dadurch gekennzeichnet, dass** der pflanzliche Faserstoff des zweiten Materials ein Holzstoff, insbesondere Holzschliff, oder Zellstoff oder Baumwollfaser oder Grasfaser ist.

11. Vorrichtung nach einem der vorhergehenden Ansprüche, **dadurch gekennzeichnet, dass** das mineralische Material Steinmehl oder Bentonit oder Silikagel ist.

12. Vorrichtung nach einem der vorhergehenden Ansprüche, **dadurch gekennzeichnet, dass** die Einlage (20) mehrere miteinander, insbesondere durch einen Klebstoff, verbundene Lagen (21) des zweiten Materials aufweist.

13. Vorrichtung nach einem der vorhergehenden Ansprüche, **dadurch gekennzeichnet, dass** das Gehäuse (10) ein erstes Gehäuseteil (13) und ein zweites Gehäuseteil (14) aufweist, wobei das erste Gehäuseteil (13) und/oder das zweite Gehäuseteil (14) eine Aufnahme für die Einlage (20) umfasst, wobei das erste Gehäuseteil (13) eine oder mehrere Durchgangsöffnungen (12) aufweist, durch welche der flüchtige Stoff in die Umgebungsluft abgegeben werden kann, und das zweite Gehäuseteil (14) aus einer Schließstellung, in welcher es die Durchgangsöffnungen des ersten Gehäuseteils (13) verdeckt, in eine Offenstellung verschiebbar ist, in welcher die Durchgangsöffnungen (12) des ersten Gehäuseteils (13) zur Abgabe des flüchtigen Stoffs freigegeben sind.

14. Vorrichtung nach einem der Ansprüche 1 bis 12, **dadurch gekennzeichnet, dass** das Gehäuse (10) ein erstes Gehäuseteil (13), ein zweites Gehäuseteil (14) und ein drittes Gehäuseteil (15) aufweist, wobei das erste Gehäuseteil (13) und/oder das zweite Gehäuseteil (14) eine Aufnahme für die Einlage (20) umfasst, wobei das dritte Gehäuseteil (15) eine oder mehrere Durchgangsöffnungen (12) aufweist, durch welche der flüchtige Stoff in die Umgebungsluft abgegeben werden kann, wobei das erste Gehäuseteil (13) und/oder das zweite Gehäuseteil (14) aus einer Schließstellung, in welcher das erste Gehäuseteil (13) und/oder das zweite Gehäuseteil (14) die Durchgangsöffnungen des dritten Gehäuseteils (15) verdeckt, in eine Offenstellung verschiebbar ist, in welcher die Durchgangsöffnungen (12) des dritten Gehäuseteils (15) zur Abgabe des flüchtigen Stoffs freigegeben sind.

15. Vorrichtung nach einem der Ansprüche 1 bis 13, **dadurch gekennzeichnet, dass** das Gehäuse (10) eine Aufnahme für die Einlage (20) und ein oder mehrere Durchgangsöffnungen (12) aufweist, durch welche der flüchtige Stoff in die Umgebungsluft abgegeben werden kann, wobei die Durchgangsöffnungen (12) mit einem entfernbaren Barriereelement (11), beispielsweise einer Folie, versehen sind.
